# EUROPÄISCHE PATENTSCHRIFT

(11) **EP 1 418 895 B1**
(45) Veröffentlichungstag und Bekanntmachung des Hinweises auf die Patenterteilung: **19.12.2012**
(21) Anmeldenummer: 02762444.4
(22) Anmeldetag: 13.08.2002
(51) Int. Cl.: A61K 9/70, A61P 5/24, A61P 15/08, A61P 15/12, A61P 5/18

(54) **TRANSDERMALES THERAPEUTISCHES SYSTEM AUF DER BASIS VON POLYACRYLAT-HAFTKLEBERN OHNE FUNKTIONELLE GRUPPEN**
TRANSDERMAL THERAPEUTIC SYSTEM BASED ON POLYACRYLATE-CONTACT-BONDING ADHESIVES WITHOUT FUNCTIONAL GROUPS
SYSTEME THERAPEUTIQUE TRANSDERMIQUE A BASE D'ADHESIFS DE CONTACT POLYACRYLATE DEPOURVUS DE GROUPES FONCTIONNELS

(30) Priorität: 24.08.2001 DE 10141652
(43) Veröffentlichungstag der Anmeldung: 19.05.2004
(73) Patentinhaber: LTS LOHMANN Therapie-Systeme AG, 56626 Andernach (DE)
(72) Erfinder: KLEIN, Robert-Peter, deceased (DE); HILLE, Thomas, 56567 Neuwied (DE); THEOBALD, Frank, 53498 Bad Breisig (DE)
(74) Vertreter: Siemund, Volker
(86) Internationale Anmeldenummer: PCT/EP2002/009057
(87) Internationale Veröffentlichungsnummer: WO 2003/017988

(56) Entgegenhaltungen:
- EP-A2- 1 103 260
- WO-A-02/24157
- US-B1- 6 210 705
- DATABASE WPI Section Ch, Week 199047 Derwent Publications Ltd., London, GB; Class A96, AN 1990-352772 XP002223370 & JP 02 255611 A (NITTO DENKO CORP), 16. Oktober 1990 (1990-10-16)

## Beschreibung

Bei transdermalen therapeutischen Systemen (TTS) handelt es sich um flächenförmige, schichtweise aufgebaute pharmazeutische Produkte, bei denen ein oder mehrere Wirkstoffe mit oder ohne Hilfsstoffe (z. B. Penetrationsbeschleuniger) in eine ggf. haftklebende Polymermatrix eingebettet sind. In der Regel wird diese Polymermatrix hergestellt, indem eine Trägerfolie mit der den Wirkstoff enthaltende Polymermasse beschichtet und anschließend mit einer Abdeckfolie versehen wird, welche auch während der Applikation des transdermalen therapeutischen Systems auf der Haut verbleibt. Die Trägerfolie dient als Schutzschicht für die Polymermatrix während der Dauer der Lagerung und gegebenenfalls als Applikationshilfe für die spätere Anwendung des transdermalen therapeutischen Systems.

Transdermale therapeutische Systeme ermöglichen eine kontinuierliche Wirkstoffzufuhr über den gesamten Applikationszeitraum. Sie sind daher bezüglich ihrer Konzentrations-Zeit-Profile vergleichbar mit Dauertropfinfusionen. Zahlreiche transdermale therapeutische Systeme mit unterschiedlichen Wirkstoffen und Wirkstoffkombinationen befinden sich heute auf dem Arzneimittelmarkt. Eines der wichtigsten Indikationsgebiete für transdermalen therapeutische Systeme ist die Hormonsubstitutionstherapie, insbesondere bei Frauen in der Menopause. In den frühen Jahren der transdermalen Hormonsubstitutionstherapie wurden hierfür vor allem estrogenhaltige Monopräparate eingesetzt. Neuerdings werden jedoch transdermale therapeutische Systeme angeboten, die eine Kombination von Estrogenen (z. B. 17β-Estradiol) und Gestagenen (z. B. Norethisteron) enthalten. Testosteron, das männliche Sexualhormon gehört ebenfalls zur Gruppe der Steroidhormone, die im Rahmen einer Hormonsubstitutionstherapie Verwendung finden, insbesondere bei der Behandlung des Hypogonadismus.

Eine Reihe von kommerziell erhältlichen transdermalen therapeutischen Systemen sind als sogenannte Matrixsysteme konstruiert. Hierbei handelt es sich um Systeme, bei denen die haftklebend oder nicht-haftklebend ausgerüstete Polymermatrix den Wirkstoff in gelöster oder suspendierter Form enthält. Die Polymermatrix besteht dabei meistens aus Haftklebern auf der Basis von Polyacrylaten.

Die dabei verwendeten Polyacrylate werden aus Monomeren (Acrylsäure und Methacrylsäure sowie jeweils deren Ester, ggf. mit Vinylacetat) hergestellt, welche funktionelle Gruppen enthalten. Diese funktionellen Gruppen können die Polymerisation der eingesetzten Monomere unverändert überstehen und beeinflussen die Eigenschaften des entstehenden Polyacrylats - insbesondere die Klebrigkeit ("tackiness") und das Haftvermögen - entscheidend.

So sind aus EP 614 356 Kleberformulierungen auf Polyacrylatbasis bekannt, bei denen der Anteil der Summe von Acrylsäure, Glycidylmethacrylat und Hydroxyethylacrylat zwischen 4,8 und 5,5 Gew.-% beträgt (vergl. Tab. 3 dieses Dokuments).

In der JP 2-255 611 A wird ein wirkstoffhaltiges Pflasterpräparat mit einem basischen Wirkstoff und einer Haftkleberschicht auf einem flexiblen Träger offenbart. Der Haftkleber ist erhältlich durch Polymerisierung eines Alkyl(meth)acrylats und/oder eines alkoxy-modifizierten Monomers. In einem konkreten transdermalen therapeutischen System mit einem Basispolymer bestehend aus 60% Acrylsäure-isooctylester und 40% Methylmethacrylat wird Scopolamin als Wirkstoff in Form einer freien Base eingearbeitet. Durch Hydroxyl- und Carboxylgruppen im Acrylpolymer das Haftklebers wird der Wirkstoff temporär festgehalten und zeitverzögert, aber quantitativ freigesetzt.

WO 02/24157 A2 offenbart eine Zusammensetzung von Haftklebern zur Verwendung in transdermalen therapeutischen Applikationen, herstellbar aus Mischungen Alkylacrylat bzw. Alkyl(meth)acrylat und polymerisierbaren, nichtcyclischen, stickstoffhaltigen Monomeren, insbesondere Vinylamide, N-substituierte Acrylamide bzw. Methacyrlamide.

US 6,210,705 B1 offenbart ein Arzneimittel und Verfahren zur Behandlung von Aufmerksamkeitsstörungen und Aufmerksamkeits-/Hyperaktivitätsstörungen, das als Wirkstoff Methylphenidat in einer topischen Applikationsform enthält. Für haftklebende Ausführungsformen werden natürliche und synthetische Polymere angegeben, unter anderem bevorzugt Haftkleber auf Polyacrylatbasis.

Der Fachmann unterscheidet Polyacrylate mit -OH-Gruppen (Hydroxylgruppen) und solche mit -COOH-Gruppen (Carboxylgruppen) als funktionellen Gruppen. Zur den hydroxylgruppenhaltigen Polyacrylaten zählt beispielsweise Durotak 2287, zu den carboxylgruppenhaltigen Polyacrylaten beispielsweise Durotak 2051, die beide von der Fa. National Starch hergestellt werden. Diese Polyacrylate haben sich als stabile und gut verträgliche haftklebende Polymere für die Herstellung von transdermalen therapeutischen Systemen, die Steroidhormone als Wirkstoffe enthalten, erwiesen.

Nachteilig bei den transdermalen therapeutischen Systemen, deren Polymermatrices Polyacrylate enthalten, welche die genannten funktionellen Gruppen (Hydroxylgruppe, Carboxylgruppe) enthalten, ist die geringe Wirkstoffausnutzung. Dies ist insbesondere bei hormonhaltigen transdermalen therapeutischen Systemen zu beobachten. Dabei ist unter einer geringen Wirkstoffausnutzung zu verstehen, dass nach Ablauf der vorgesehenen Applikationsdauer des transdermalen therapeutischen Systems eine im Vergleich zur der vor Beginn der Verabreichung dieses transdermalen therapeutischen Systems darin enthaltenen Gesamtmenge des Wirkstoffs eine relativ große Menge des Wirkstoffs ungenutzt im "verbrauchten" transdermalen therapeutischen System zurückbleibt.

Da zum Teil sehr teure Wirkstoffe in transdermalen therapeutischen Systemen eingesetzt werden, ist die geringe Wirkstoffausnutzung aus ökonomischen und aus ökologischen Gesichtspunkten unerwünscht. Schließlich kann bei in höherer Konzentration toxisch wirkenden pharmazeutischen Wirkstoffen ein hoher Restgehalt auch ein gewisses Gefährdungspotential für eine missbräuchliche Einnahme einer höheren Dosis darstellen.

Erfindungsgemäß wird dieser Nachteil durch ein transdermales therapeutisches System gelöst, das als Basispolymer für die Polymermatrix ein haftklebendes Polyacrylat enthält, welches einen extrem reduzierten Anteil von Hydroxylgruppen und / oder Carboxylgruppen enthält, so dass dieses als "im wesentlichen frei von funktionellen Gruppen" bezeichnet werden kann. Dies ist umso überraschender, da in den Fachkreisen das Vorhandensein von funktionellen Gruppen, insbesondere Hydroxylgruppen und / oder Carboxylgruppen in den Polyacrylaten als Voraussetzung für eine gute Kohäsion und / oder Adhäsion der Polymermatrix betrachtet wird.

Als Polyacrylate, welche erfindungsgemäß "im wesentlichen frei von funktionellen Gruppen" sind, kommen Polymere (Homopolymere, Copolymere und Block-Copolymere) auf Basis von Acrylsäureestern und / oder Methacrylsäureestern in Frage.

Als Monomere zur Herstellung des erfindungsgemäßen Polyacrylats kommen dabei insbesondere n-Butylacrylat, n-Butylmethacrylat, Ethylacrylat, 2-Ethylhexylacrylat, Ethylmethacrylat, Methylacrylat, Methylmethacrylat, tert.-Butylacrylat, sec.-Butylacrylat, tert.-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen dieser Monomere in Frage. Es handelt sich bei diesen Monomeren um Ester der Acryl- bzw. Methacrylsäure, die lineare, verzweigte oder cyclische aliphatische C₁-C₁₂-Substituenten ohne sonstige funktionelle Gruppen tragen.

Auch kann Vinylacetat als Co-Monomer zusammen mit mindestens einem dieser Monomere zur Herstellung des Polyacrylats verwendet werden. Der Anteil des Vinylacetats in dem zur Herstellung des Polyacrylats verwendeten Monomerengemisch sollte unterhalb von 20 Gew.-%, vorzugsweise unterhalb von 5 Gew.-% liegen. Besonders bevorzugt ist ein Vinylacetatgehalt unterhalb von 1,5 Gew.-%.

Unter den Estern der Acrylsäure bzw. Methacrylsäure, welche funktionelle Gruppen tragen und in dem zur Herstellung des Polyacrylats verwendeten Monomerengemisch enthalten sein können, sind in erster Linie hydroxylgruppenhaltige Ester zu verstehen, also 2-Hydroxyethylacrylat, 2-Hydroxyethylmethacrylat, 3-Hydroxypropylacrylat und 3-Hydroxypropylmethacrylat. Aber auch Stoffe wie Acrylnitril, Methacrylnitril, Acrylamid, Dimethylaminoethylacrylat etc. können im Sinne dieser Beschreibung als "Ester der Acrylsäure bzw. Methacrylsäure mit funktionellen Gruppen" betrachtet werden.

Allerdings ist der Anteil der Summe von Acrylsäure, Methacrylsäure, 2-Hydroxyethylacrylat, 2-Hydroxyethylmethacrylat, 3-Hydroxypropylacrylat und /oder 3-Hydroxypropylmethacrylat im dem zur Herstellung des Polyacrylats verwendeten Monomerengemisch unterhalb von 2 Gew.-%, vorzugsweise unterhalb von 1,5 Gew.-% und besonders bevorzugt unterhalb von 0,2 Gew.-%.

Unter "im wesentlichen frei von funktionellen Gruppen" ist im Sinne der vorliegenden Beschreibung also zu verstehen, dass der Gesamtanteil von Acrylsäure, Methacrylsäure und Estern der Acrylsäure bzw. Methacrylsäure, welche funktionelle Gruppen tragen (insbesondere die hydroxylgruppenhaltigen Ester), im Polyacrylat unterhalb von 2 Gew.-%, vorzugsweise unterhalb von 1,5 Gew.-% liegt. In einer besonderen Ausführungsform liegt der Gesamtanteil dieser Monomere unterhalb von 0,2 Gew.-%. In einer besonderen Ausführungsform sind keine dieser Ester der Acrylsäure bzw. Methacrylsäure, welche funktionelle Gruppen tragen, im Monomerengemisch enthalten.

Die Monomerengemische können auf verschiedene Art polymerisiert werden, z. B. ionisch, radikalisch, lichtinduziert etc., gegebenenfalls unter Verwendung von Vemetzem wie z. B. Aluminiumacetylacetonat, Allylglycidylether und / oder Glycidylmethacrylat (die - sofern vorhanden - in einem Anteil unterhalb von 0,5 Gew.-% im Monomerengemisch enthalten sind) und gegebenenfalls auch unter Verwendung von Hilfsstoffen wie Antioxidantien, Stabilisatoren und / oder Alkylmercaptanen (die - sofern vorhanden - in einem Anteil unterhalb von 0,1 Gew.-% im Monomerengemisch enthalten sind). Es können Wasser, ggf. zusammen mit Emulgatoren oder organische Lösungsmittel als Reaktionsmedium verwendet werden.

Haftkleber auf Basis von Polyacrylaten, die nach unserer Ansicht unter diese Definition von "im wesentlichen frei von funktionellen Gruppen" fallen, sind der Elite-Kleber der Fa. National Starch sowie GMS 3083 der Fa. Solutia.

In einer besonders einfachen Ausführungsform besteht die Polymermatrix ausschließlich aus dem Wirkstoff (bzw. einer Wirkstoffkombination) und dem erfindungsgemäßen Polyacrylat. Es sind aber auch Ausführungsformen möglich, bei denen eine Mischung eines Polyacrylats ohne funktionelle Gruppe mit einem Polyacrylat mit funktionellen Gruppen verwendet wird.

Als Wirkstoffe kommen Steroidhormone in Frage,die als pharmazeutisch wirksame Stoffe in den Polymermatrices auf Basis von Polyacrylaten ohne funktionelle Gruppen verwendet werden. Hierzu zählen: Androgene wie z. B. Boldenon, Fluoxymesteron, Mestanolon, Mesterolon, Methandrostenolon, 17-Methyltestosteron, 17α-Methyl-testosteron-3-cyclopentylenolether, Norethandrolon, Normethandron, Oxandrolon, Oxymetholon, Prasteron, Stanolon, Stanozolol, Testosteron, Testosteron-17-chloral Halbacetal, Testosteron 17β-cypionat, Testosteronenanthat, Testosteronnicotinat, Testosteronphenylacetat, Testosteronpropionat, Tiomesterone,

Estrogene wie z. B. Benzestrol, Broparoestrol, Chlorotrianisen, Dienestrol, Diethylstilbestrol, Diethylstilbestroldipropionat, Dimestrol, Fosfestrol, Hexestrol, Methallenestril, Methestrol, Colpormon, Equilenin, Equilin, konjugierte estrogene Hormone, Estrogenester, Estropipat, 17β-Estradiol, Estradiol, Estradiolbenzoat, Estradiol-17β-cypionat, Estriol, Estron, Ethinylestradiol, Mestranol, Moxestrol, Mytatrienediol, Polyestradiolphosphat, Quinestradiol, Quinestrol,
Gestagene wie z. B. Allylestrenol, Anageston, Chlormadinonacetat, Delmadinonacetat, Demegeston, Desogestrel, Dimethisteron, Dydrogesteron, Ethinylestrenol, Ethisteron, Ethynodiol, Ethynodioldiacetat, Flurogestonacetat, Gestoden, Gestonoroncaproat, Haloprogesterone, 17-Hydroxy-16-methylenprogesteron, 17α-Hydroxyprogesteron, 17α-Hydroxygesteroncaproat, Levonorgestrel, Lynestrenol, Medrogeston, Medroxyprogesteron, Megestrolacetat, Melengestrol, Norethindron, Norethindronacetat, Norethynodrel, Norgesteron, Norgestimate, Norgestrel, Norgestrienon, 19-Norprogesteron, Norvinisteron, Pentagestron, Progesteron, Promegeston, Quingestron und Trengeston.

### Beispiel

Es wurden vier transdermale therapeutische Systeme (Formulierungen Nr. 1 bis 4) hergestellt, die als Wirkstoffe 17β-Estradiol bzw. Testosteron in der Polymermatrix enthielten. Als Rückschicht wurde eine PolyethylenterephthalatFolie verwendet, das Flächengewicht der Polymermatrix betrug 100 g/m². (Die Dicke der Polymermatrix kann vorzugsweise zwischen 15 bis 30 µm betragen.)

Zum Vergleich des Verhaltens hinsichtlich der Wirkstoffausnutzung wurde einerseits für die Polymermatrix Durotak 2287 als haftklebendes Polyacrylat mit funktioneller Gruppe eingesetzt, während in den erfindungsgemäßen transdermalen therapeutischen Systemen der Kleber GMS 3083 der Fa. Solutia als ein im Sinne dieser Beschreibung haftklebendes Polyacrylat ohne funktionelle Gruppe Verwendung fand.

In den Tabellen 1 und 2 sind die kumulierten Wirkstoffmengen für die jeweiligen Polymermatrices dargestellt, welche in einer Franz'schen Zelle, die mit einer EVA-Membran ausgerüstet war, gemessen wurden.

**Tabelle 1: Freisetzungsverhalten aus estradiolhaltigen Polymermatrices**

| **Nr.** | **Inhaltsstoffe** | **%-Gehalt** | **Kumulierte Wirkstoffmenge in [µg/cm²] (nach 3 Tagen)** |
|---|---|---|---|
| 1 | 17β-Estradiol | 1,00 | 170,3 |
| | Durotak 2287 | 99,00 | |
| 2 | 17β-Estradiol | 1,00 | 240,6 |
| | GMS 3083 | 99,00 | |

**Tabelle 2: Freisetzung aus testosteronhaltigen Polymermatrices**

| **Nr.** | **Inhaltsstoffe** | **%-Gehalt** | **Kumulierte Wirkstoffmenge in [µg/cm²] (nach 3 Tagen)** |
|---|---|---|---|
| 3 | Testosteron | 2,00 | 575,3 |
| | Durotak 2287 | 98,00 | |
| 4 | Testosteron | 2,00 | 675,5 |
| | GMS 3083 | 98,00 | |

Die Wirkstoffausnutzung ergibt sich aus der Kumulierten Wirkstoffmenge dividiert durch die im TTS enthaltene Wirkstoffmenge.

Wie man sieht, konnte im Falle des Estradiols durch Verwendung des Polyacrylatklebers ohne funktionelle Gruppen eine 40%-ig bessere Wirkstoffausnutzung, im Falle des Testosterons eine 17%-ig bessere Wirkstoffausnutzung erzielt werden. Mit anderen Worten: Der kumulative Flux liegt um den Faktor 40% bzw. 17% höher. So kann bei gleicher Wirkstoffbeladung eine bessere Wirkstoffausnutzung erzielt werden.

## Patentansprüche

1. Transdermales therapeutisches System umfassend eine Rückschicht, eine Schutzschicht und mindestens eine wirkstoffhaltige Polymermatrix, wobei der Wirkstoff ein Steroidhormon oder eine Kombination von Steroidhormonen ist, und wobei das Basispolymer der Polymermatrix aus einem haftklebenden Polyacrylat besteht, das ein Homopolymer, Copolymer oder Block-Copolymer ist, welches herstellbar ist durch Polymerisation eines Monomerengemisches bestehend aus:
a) einem Monomeren oder einer Mischung von Monomeren aus der Gruppe der Ester der Acryl- bzw. Methacrylsäure, die lineare, verzweigte und / oder cyclische aliphatische C₁-C₁₂-Substituenten ohne sonstige funktionelle Gruppe tragen,
b) gegebenenfalls Acrylsäure, Methylacrylsäure, 2-Hydroxyethylacrylat, 2-Hydroxyethylmethacrylat, 3-Hydroxypropylacrylat und / oder 3-Hydroxypropylmethacrylat, wobei der Anteil dieser Monomere in der Summe unterhalb von 2 Gew.-% liegt,
c) gegebenenfalls Vinylacetat in einem Anteil unterhalb von 20 Gew.-%,
d) gegebenenfalls Vernetzer in einem Anteil unterhalb von 0,5 Gew.-%
e) gegebenenfalls Hilfsstoffen aus der Gruppe der Antioxidantien, Stabilisatoren und / oder Alkylmercaptanen in einem Anteil unterhalb von 0,1 Gew.-%.

2. Transdermales therapeutisches System nach Anspruch 1, **dadurch gekennzeichnet, dass** die Polymerisation des Monomerengemisches ionisch, radikalisch oder lichtinduziert in Wasser, welches Emulgatoren enthalten kann oder in organischen Lösungsmitteln als Reaktionsmedium erfolgt.

3. Transdermales therapeutisches System nach Anspruch 1 oder 2, **dadurch gekennzeichnet, dass** das Monomerengemisch Vinylacetat in einem Anteil unterhalb von 5 Gew.-% enthält.

4. Transdermales therapeutisches System nach einem oder mehreren der Ansprüche 1 bis 3, **dadurch gekennzeichnet, dass** im Monomerengemisch als Vernetzer Aluminiumacetylacetonat, Allylglycidylether und / oder Glycidylmethacrylat verwendet wird.

5. Transdermales therapeutisches System nach einem oder mehreren der Ansprüche 1 bis 4, **dadurch gekennzeichnet, dass** die Gruppe der Ester der Acryl- bzw. Methacrylsäure, die lineare, verzweigte und / oder cyclische aliphatische C₁-C₁₂-Substituenten ohne sonstige funktionelle Gruppe enthalten, aus n-Butylacrylat, n-Butylmethacrylat, Ethylacrylat, 2-Ethylhexylacrylat, Ethylmethacrylat, Methylacrylat, Methylmethacrylat, tert.-Butylacrylat, sec.-Butylacrylat, tert.-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen dieser Monomeren besteht.

6. Transdermales therapeutisches System nach einem oder mehreren der Ansprüche 1 bis 5, **dadurch gekennzeichnet, dass** das Monomerengemisch frei von Acrylsäure, Methacrylsäure, 2-Hydroxyethylacrylat, 2-Hydroxyethylmethacrylat, 3-Hydroxypropylacrylat und / oder 3-Hydroxypropylmethacrylat ist.

7. Transdermales therapeutisches System nach einem oder mehreren der Ansprüche 1 bis 6, **dadurch gekennzeichnet, dass** das Steroidhormon 17ß-Estradiol, Ethinylestradiol, Estradiolacetat, Levonorgestrel, Norethindron, Norethindronacetat oder Testosteron ist.

8. Transdermales therapeutisches System nach einem der Ansprüche 1 bis 7 zur Anwendung in der Behandlung des Hypogonadismus, zur Hormonsubstitutionstherapie oder zur Kontrazeption.

## Claims

1. Transdermal therapeutic system comprising one back layer, one protective layer and at least one active-compound-containing polymer matrix, the active compound being a steroid hormone or a combination of steroid hormones, and the base polymer of the polymer matrix consisting of a contact-adhesive polyacrylate, which is a homopolymer, copolymer or block copolymer which can be prepared by polymerization of a monomer mixture consisting of:
a) a monomer or a mixture of monomers from the group consisting of the esters of acrylic or methacrylic acid, which carry linear, branched and/or cyclic aliphatic C₁-C₁₂ substituents without another functional group,
b) optionally acrylic acid, methacrylic acid, 2-hydroxyethyl acrylate, 2-hydroxyethyl methacrylate, 3-hydroxypropyl acrylate and/or 3-hydroxypropyl methacrylate, the content of these monomers in the total being below 2% by weight,
c) optionally vinyl acetate in a content of below 20% by weight,
d) optionally crosslinkers in a content of below 0.5% by weight
e) optionally auxiliaries from the group consisting of the antioxidants, stabilizers and/or alkylmercaptans in a content of below 0.1% by weight.

2. Transdermal therapeutic system according to Claim 1, **characterized in that** the polymerization of the monomer mixture is carried out by ionic, free-radical or light-induced means, in water which can contain emulsifiers, or in organic solvents as a reaction medium.

3. Transdermal therapeutic system according to Claim 1 or 2, **characterized in that** the monomer mixture contains vinyl acetate in a content of below 5% by weight.

4. Transdermal therapeutic system according to one or more of Claims 1 to 3, **characterized in that** aluminum acetylacetonate, allyl glycidyl ether and/or glycidyl methacrylate is used in the monomer mixture as a crosslinker.

5. Transdermal therapeutic system according to one or more of Claims 1 to 4, **characterized in that** the group of esters of acrylic or methacrylic acid which contain linear, branched and/or cyclic aliphatic C₁-C₁₂ substituents without another functional group consists of n-butyl acrylate, n-butyl methacrylate, ethyl acrylate, 2-ethylhexyl acrylate, ethyl methacrylate, methyl acrylate, methyl methacrylate, tert-butyl acrylate, sec-butyl acrylate, tert-butyl methacrylate, cyclohexyl methacrylate, 2-ethylhexyl methacrylate, isobornyl methacrylate, isobutyl methacrylate, isopropyl acrylate, isopropyl methacrylate and mixtures of these monomers.

6. Transdermal therapeutic system according to one or more of Claims 1 to 5, **characterized in that** the monomer mixture is free of acrylic acid, methacrylic acid, 2-hydroxyethyl acrylate, 2-hydroxyethyl methacrylate, 3-hydroxypropyl acrylate and/or 3-hydroxypropyl methacrylate.

7. Transdermal therapeutic system according to one or more of Claims 1 to 6, **characterized in that** the steroid hormone is 17β-estra-diol, ethynylestradiol, estradiol acetate, levonorgestrel, norethindrone, norethindrone acetate or testosterone.

8. Transdermal therapeutic system according to one of Claims 1 to 7 for use in the treatment of hypogonadism, for hormone substitution therapy or for contraception.

## Revendications

1. Système thérapeutique transdermique comprenant une couche arrière, une couche de protection et au moins une matrice polymère contenant un agent actif, l'agent actif étant une hormone stéroïdienne ou une combinaison d'hormones stéroïdiennes, et le polymère de base de la matrice polymère étant constitué d'un polyacrylate adhésif de contact qui est un homopolymère, un copolymère ou un copolymère séquencé, qui peut être fabriqué par polymérisation d'un mélange de monomères constitué par :
a) un monomère ou un mélange de monomères du groupe constitué par les esters de l'acide acrylique ou méthacrylique qui portent des substituants en C₁-C₁₂ aliphatiques linéaires, ramifiés et/ou cycliques, sans autre groupe fonctionnel,
b) éventuellement l'acide acrylique, l'acide méthylacrylique, l'acrylate de 2-hydroxyéthyle, le méthacrylate de 2-hydroxyéthyle, l'acrylate de 3-hydroxypropyle et/ou le méthacrylate de 3-hydroxypropyle, la proportion de ces monomères étant au total inférieure à 2 % en poids,
c) éventuellement l'acétate de vinyle en une proportion inférieure à 20 % en poids,
d) éventuellement des agents de réticulation en une proportion inférieure à 0,5 % en poids,
e) éventuellement des adjuvants du groupe constitué par les antioxydants, les stabilisateurs et/ou les alkylmercaptans en une proportion inférieure à 0,1 % en poids.

2. Système thérapeutique transdermique selon la revendication 1, **caractérisé en ce que** la polymérisation du mélange de monomères a lieu par voie ionique, radicalaire ou induite par la lumière, dans de l'eau qui peut contenir des émulsifiants ou dans des solvants organiques en tant que milieu réactionnel.

3. Système thérapeutique transdermique selon la revendication 1 ou 2, **caractérisé en ce que** le mélange de monomères contient de l'acétate de vinyle en une proportion inférieure à 5 % en poids.

4. Système thérapeutique transdermique selon une ou plusieurs des revendications 1 à 3, **caractérisé en ce que** de l'acétylacétonate d'aluminium, de l'éther allylglycidylique et/ou du méthacrylate de glycidyle sont utilisés dans le mélange de monomères en tant qu'agent de réticulation.

5. Système thérapeutique transdermique selon une ou plusieurs des revendications 1 à 4, **caractérisé en ce que** le groupe des esters de l'acide acrylique ou méthacrylique qui portent des substituants en C₁-C₁₂ aliphatiques linéaires, ramifiés et/ou cycliques, sans autre groupe fonctionnel, est constitué par l'acrylate de n-butyle, le méthacrylate de n-butyle, l'acrylate d'éthyle, l'acrylate de 2-éthylhexyle, le méthacrylate d'éthyle, l'acrylate de méthyle, le méthacrylate de méthyle, l'acrylate de tert.-butyle, l'acrylate de sec.-butyle, le méthacrylate de tert.-butyle, le méthacrylate de cyclohexyle, le méthacrylate de 2-éthylhexyle, le méthacrylate d'isobornyle, le méthacrylate d'isobutyle, l'acrylate d'isopropyle, le méthacrylate d'isopropyle et les mélanges de ces monomères.

6. Système thérapeutique transdermique selon une ou plusieurs des revendications 1 à 5, **caractérisé en ce que** le mélange de monomères est exempt d'acide acrylique, d'acide méthacrylique, d'acrylate de 2-hydroxyéthyle, de méthacrylate de 2-hydroxyéthyle, d'acrylate de 3-hydroxypropyle et/ou de méthacrylate de 3-hydroxypropyle.

7. Système thérapeutique transdermique selon une ou plusieurs des revendications 1 à 6, **caractérisé en ce que** l'hormone stéroïdienne est le 17β-estradiol, l'éthinylestradiol, l'acétate d'estradiol, le lévonorgestrel, la noréthindrone, l'acétate de noréthindrone ou la testostérone.

8. Système thérapeutique transdermique selon l'une quelconque des revendications 1 à 7, destiné à être utilisé pour le traitement de l'hypogonadisme, pour le traitement hormonal de substitution ou pour la contraception.
